# EUROPEAN PATENT APPLICATION

(11) **EP 2 660 321 A1**
(43) Date of publication of application: **06.11.2013**
(21) Application number: 11853923.8
(22) Date of filing: 14.12.2011
(51) Int. Cl.: C12N 15/09, C12Q 1/68

(54) **METHOD FOR DETECTING METHYLATED CYTOSINE**

(30) Priority: 27.12.2010 JP 2010289071
(71) Applicant: Wako Pure Chemical Industries, Ltd., Osaka-shi Osaka 540-8605 (JP)
(72) Inventor: HAYASHIDA Yukinobu, Amagasaki-shi Hyogo 661-0963 (JP)
(74) Representative: Winter, Brandl, Fürniss, Hübner, Röss, Kaiser, Polte - Partnerschaft
(86) International application number: PCT/JP2011/078848
(87) International publication number: WO 2012/090705

(57) **Abstract**

It is an object of the present invention is to provide a method for obtaining DNA after bisulfite reaction which can be stored with libraries in genome reserved and has excellent storage stability, and to provide a method for detecting methylated cytosine more accurately as compared with the conventional bisulfite reaction. Specifically, the present invention relates to "a method for obtaining DNA complementary to the single-stranded DNA in which non-methylated cytosine has been uracilated, comprising subjecting the single-stranded DNA to 1) bisulfite reaction and 2) reverse transcriptase reaction in this order", "a method for amplifying DNA complementary to the single-stranded DNA in which non-methylated cytosine has been converted to uracil, comprising subjecting the single-stranded DNA to 1) bisulfite reaction, 2) reverse transcriptase reaction and 3) PCR reaction in this order", and "a method for detecting methylated cytosine in the single-stranded DNA, comprising subjecting the single-stranded DNA to 1) bisulfite reaction, 2) reverse transcriptase reaction 3) PCR reaction in this order, and the obtained PCR amplification product is subjected to nucleotide sequence analysis".

## Description

### TECHNICAL FIELD

The present invention relates to a method for detecting methylated cytosine in DNA using conversion of non-methylated cytosine into uracil by bisulfite reaction.

### BACKGROUND ART

It has been known that methylation of genomic DNA in a living organism is caused to suppress expression of mRNA. Further, it has been reported that the difference of methylation pattern on a genome relates to genesis, differentiation, and disease such as cancer, and therefore the analysis of methylation of genomic DNA has an important role in finding out the cause and prevention of disease, development of medicinal products, research on the regenerative medicine, and so on.
On the other hand, as the method for determining methylated cytosine in DNA nucleotide sequence, a method for comparing the fragments by methylation-sensitive restriction enzyme, a bisulfite method, a methylation-specific PCR method, and a method which utilizes a high performance liquid chromatography (HPLC), etc have been known. Among them, the bisulfite method has become popular as a common method because the bisulfite method is low cost and applicable to high throughput, and is also effective for sequencing and screening.
However, since conversion rate from non-methylated cytosine into uracil is not high in said bisulfite method, the bisulfite method had problems of low accuracy etc. in detection of methylated cytosine. Therefore, until now, development of a bisulfite method having high accuracy has been desired.
In addition, the DNA which had been subjected to the bisulfite reaction was inferior in storage stability because non-methylated cytosine is converted to uracil. That is, even if the gene which was subjected to the bisulfite reaction was intended to use again, the period during which the gene can be stored stably was only about several days. Therefore, in order to utilize it again, usually the one amplified by the PCR had to be stored. However, since only specific DNA was amplified in the one subjected to the PCR reaction, it was not suitable for storing with libraries in the genome reserved. Therefore, the development of a method for obtaining a DNA after bisulfite reaction, which can be stored with libraries in the genome reserved is excellent in storage stability, has also been desired.

### SUMMARY OF THE INVENTION

### PROBLEM TO BE SOLVED BY THE INVENTION

It is an object of the present invention to provide a method for obtaining a DNA after bisulfite reaction, which can be stored with libraries in genome reserved and has excellent storage stability, and also to provide a method for detecting methylated cytosine more accurately as compared with the conventional bisulfite reaction.

### MEANS FOR SOLVING THE PROBLEM

In view of the above-described situation, the present inventors have investigated extensively to develop a bisulfite method having high accuracy. As a result, the present inventors have found that a single-stranded DNA obtained by subjecting the single-stranded DNA which has been subjected to the bisulfite reaction to reverse transcriptase reaction was excellent in storage stability due to not including uracil., and thus the present invention has been achieved. In addition, since the said single-stranded DNA is a solution before the PCR reaction, libraries in the gene was reserved. Therefore, as described above, the single-stranded DNA obtained by subjecting single-stranded DNA to the bisulfite reaction and to the reverse transcriptase reaction was excellent in storage stability and also was kept the library reserved, which solved the problems of the conventional method. Usually, the reverse transcriptase reaction is not carried out using DNA as a template, and therefore, as described above, the subjecting the bisulfite-reacted DNA to the reverse transcriptase reaction as a template was the first trial carried out by the present inventors. Further, it was unexpected that such effect could be acquired by the trial.
Furthermore, it was found that, by subjecting the reaction product obtained by subjecting the single-stranded DNA to the bisulfite reaction and reverse transcriptase reaction, to the PCR, the single-stranded DNA, in which non-methylated cytosine has been uracilated, could be amplified efficiently, and thus attained the present invention.

That is, the present invention relates to "a method for obtaining DNA complementary to a single-stranded DNA in which non-methylated cytosine has been uracilated, comprising subjecting the single-stranded DNA to 1) bisulfite reaction and 2) reverse transcriptase reaction in this order (hereinafter, sometimes abbreviated as a method for obtaining complementary DNA of uracilated DNA of the present invention)", "a method for amplifying DNA complementary to a single-stranded DNA in which non-methylated cytosine has been uracilated, comprising subjecting the single-stranded DNA to 1) bisulfite reaction, 2) reverse transcriptase reaction and 3) PCR reaction in this order (hereinafter, sometimes abbreviated as a complementary DNA amplifying method of uracilated DNA of the present invention)", and "a method for detecting methylated cytosine in a single-stranded DNA, comprising subjecting the single-stranded DNA to 1) bisulfite reaction, 2) reverse transcriptase reaction 3) PCR reaction in this order, and subjecting the obtained PCR amplification product to nucleotide sequence analysis (hereinafter, sometimes abbreviated as a method for detecting methylated cytosine of the present invention)".

### EFFECT OF THE INVENTION

The DNA obtained by the method for obtaining complementary DNA of uracilated DNA of the present invention is excellent in storage stability due to not having uracil, and can be stored with library in the gene reserved. In addition, according to the method of the present invention, DNA can be amplified with converting almost of all non-methylated cytosine into uracil efficiently. As a consequence, detection of methylated cytosine can be performed with a high accuracy.

### BRIEF DESCRIPTION OF THE DRAWINGS

[Fig. 1]

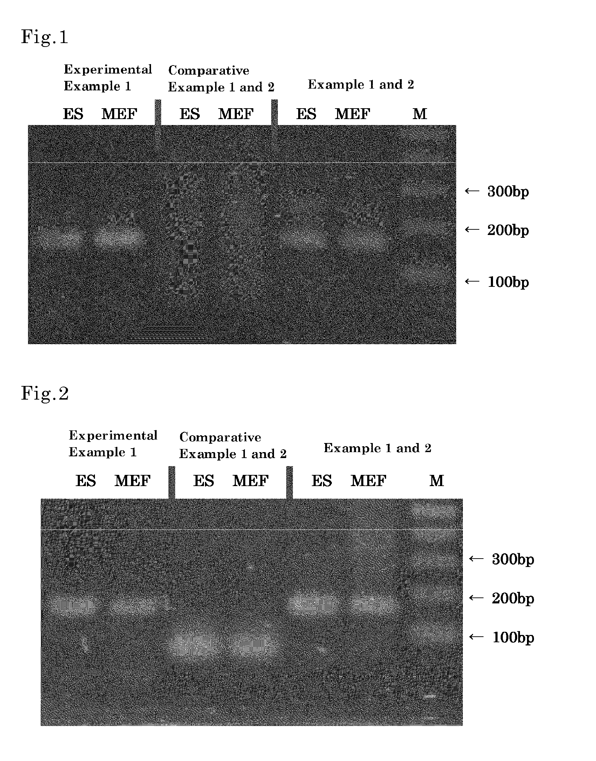

   Figure 1 is the result of electrophoresis carried out for various kinds of PCR amplified products. The figure shows, in order from the left, the results of electrophoresis of *Nanog* gene derived from ES cell and MEF cell in Experimental Example 1, *Nanog* gene derived from ES cell in Comparative Example 1, *Nanog* gene derived from MEF cell in Comparative Example 2, *Nanog* gene derived from ES cell in Example 1, and *Nanog* gene derived from MEF cell in Example 2, respectively, and the right end shows a marker [(one Step Ladder 100, 0.1-2 kbp (produced by Nippon Gene Co., Ltd.)].
[Fig. 2]
   Figure 2 is the result in which various kinds of PCR amplified products were subjected to electrophoresis. The figure shows, in order from the left, the results of electrophoresis of *Rex1* gene derived from ES cell and MEF cell in Experimental Example 1, *Rex1* gene derived from ES cell in Comparative Example *1, Rex1* gene derived from MEF cell in Comparative Example 2, *Rex1* gene derived from ES cell in Example 1, and *Rex1* gene derived from MEF cell in Example 2, respectively, and the right end shows a marker [(one Step Ladder 100, 0.1-2 kbp (produced by Nippon Gene Co., Ltd.)].
[Fig. 3]

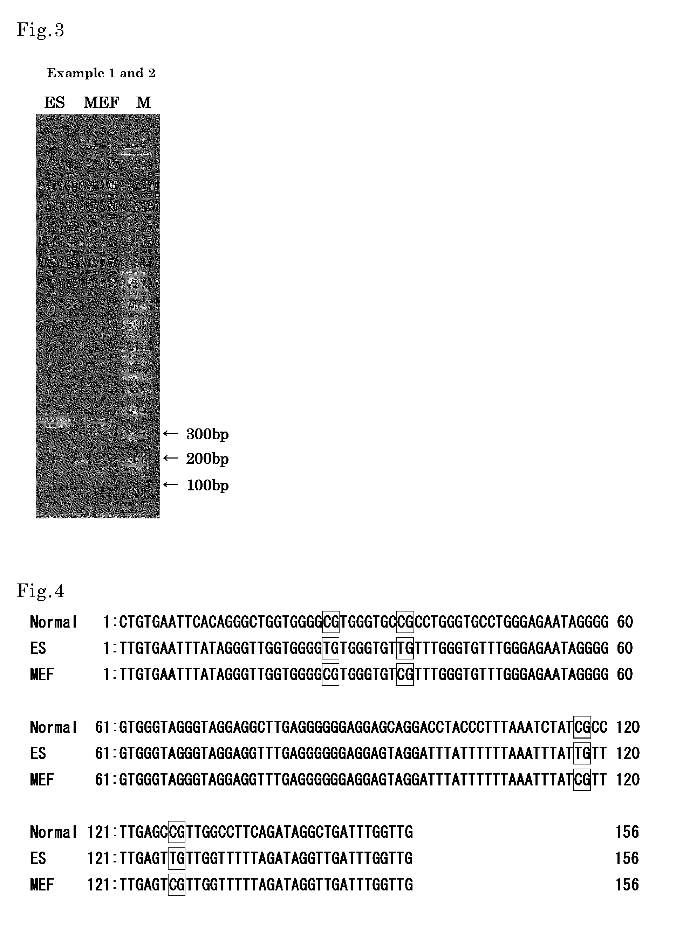

   Figure 3 shows, in order from the left, the results of electrophoresis of *CD133* gene derived from ES cell in Example 1, and *CD133* gene derived from MEF cell in Example 2, respectively, and the right end shows a marker [(one Step Ladder 100, 0.1-2 kbp (produced by Nippon Gene Co., Ltd.)].
[Fig. 4]
   In Figure 4, "Normal" represents a known nucleotide sequence of *Nanog* gene (Nucleotide Sequence 13, GenBank Accession No. AC131715), "ES" represents a nucleotide sequence of *Nanog* gene (Nucleotide Sequence 14) derived from ES cell obtained by the method for detecting methylated cytosine of the present invention, and "MEF" represents a nucleotide sequence of *Nanog* gene (Nucleotide Sequence 15) derived from MEF cell obtained by the method for detecting methylated cytosine of the present invention, respectively.
[Fig. 5]

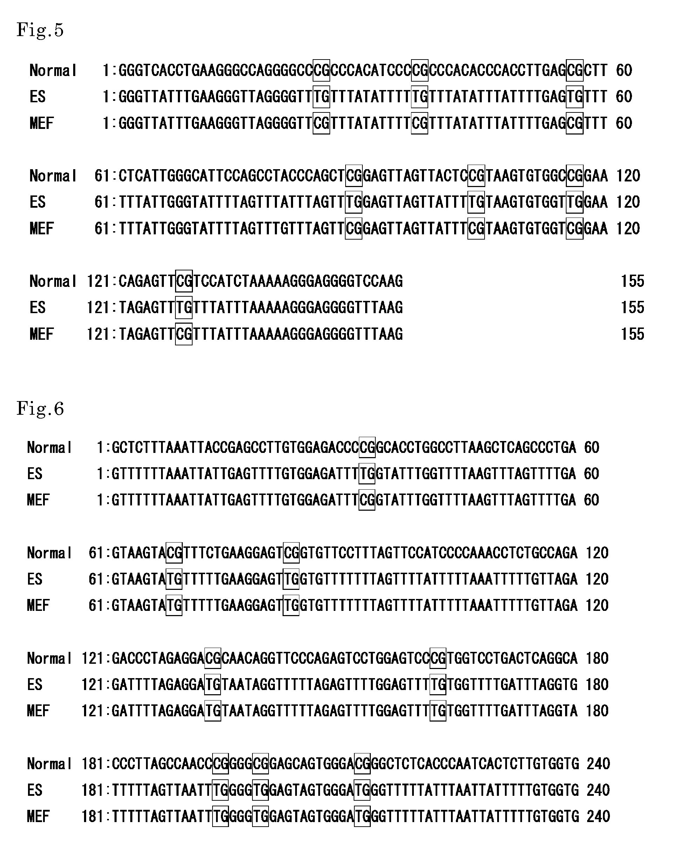

   In Figure 5, "Normal" represents a known nucleotide sequence of *Rex1* gene (Nucleotide Sequence 16, GenBank Accession No. AC127575), "ES" represents a nucleotide sequence of *Rex1* gene (Nucleotide Sequence 17) derived from ES cell obtained by the method for detecting methylated cytosine of the present invention, and "MEF" represents a nucleotide sequence of *Rex1* gene (Nucleotide Sequence 18) derived from MEF cell obtained by the method for detecting methylated cytosine of the present invention, respectively.
[Fig. 6]
   In Figure 6, "Normal" represents a known nucleotide sequence of *CD133* gene (Nucleotide Sequence 19, GenBank Accession No. AC103621), "ES" represents a nucleotide sequence of *CD133* gene (Nucleotide Sequence 20) derived from ES cell obtained by the method for detecting methylated cytosine of the present invention, and "MEF" represents a nucleotide sequence of *CD133* gene (Nucleotide Sequence 21) derived from MEF cell obtained by the method for detecting methylated cytosine of the present invention, respectively.

### DETAILED DESCRIPTION OF THE INVENTION

As a single-stranded DNA pertaining to the present invention, a single-stranded DNA containing methylated cytosine is preferable, and the one, which has a promoter region where the content rate of methylated cytosine is high, is preferable. The single-stranded DNA includes unknown sequence and known sequence. In the case of unknown sequence, the nucleotide sequence of the single-stranded DNA before being subjected to the bisulfite reaction has to be analyzed, and therefore, the known sequences is more preferable. The number of base of said single-stranded DNA is usually 50 to 300 bases, preferably 80 to 300 bases, and more preferably it is 100 to 200 bases.

The single-stranded DNA pertaining to the present invention can be obtained, according to well-known DNA extraction methods such as alkaline SDS method described in, for example, "Labo Manual for Genetic Engineering" (Maruzen Co., Ltd.) and "Handbook of Gene Technology" (Yodosha Co., Ltd.) etc., alternatively, by extracting from a cell, a microorganism, a virus, and the like using a commercially available extraction kit of genomic DNA. It should be noted that, in the case where the extracted DNA is double stranded, single-stranded DNA can be obtained by the well known per se single strand formation treatment.

Said single strand formation treatment is not limited specifically as long as it is the treatment forming a single strand to be used usually in this field. For example, there are included a heat treatment performed at usually 80 to 100°C, preferably at 80 to 90°C for usually 30 seconds to 10 minutes, preferably for 1 to 3 minutes, or an alkaline treatment performed by contacting the DNA with alkaline circumstances, etc. Among the above, the alkaline treatment is preferable because the possibility of single-stranded DNA going back to double-stranded is low on the occasion of shifting to the next process. Said alkaline treatment is carried out, specifically for example, by adding alkali or its aqueous solution to the double-stranded DNA or a solution containing the double-stranded DNA to make the solution alkaline of usually pH 10 to pH 14, preferably pH 12 to pH 14. Said alkali includes, for example, alkali metal hydroxide such as sodium hydroxide and potassium hydroxide; alkaline-earth metal hydroxide such as barium hydroxide, magnesium hydroxide, and calcium hydroxide; alkaline metal carbonate such as sodium carbonate; ammonia, and amines and the like. Among them, alkali metal hydroxide such as sodium hydroxide and potassium hydroxide is preferable, and among these, sodium hydroxide is particularly preferable. Said alkaline treatment is performed, more specifically, by adding usually 0.1 to 1 µL, preferably 0.1 to 0.5 µL of 0.5 to 3 mmol/L aqueous alkaline solution to 1 µL of solution including single-stranded DNA, and heating usually for 5 minutes to 60 minutes, preferably for 5 to 30 minutes at usually 25 to 70°C, preferably at 30 to 50°C.

### [A method for obtaining DNA which is complementary to the single-stranded DNA in which non-methylated cytosine has been uracilated (a method for obtaining complementary DNA of uracilated DNA of the present invention)]

The method for obtaining complementary DNA of uracilated DNA of the present invention is performed by subjecting a single-stranded DNA to 1) bisulfite reaction and 2) reverse transcriptase reaction in sequence. By said method, the DNA, which is complementary to the above-described single-stranded DNA in which non-methylated cytosine has been uracilated, can be obtained easily.

The above-described bisulfite reaction includes any bisulfite reaction as long as it is the bisulfite reaction which remains methylated cytosine as it is but converts only non-methylated cytosine into uracil and which is usually used in this field. Specifically, for example, the reaction is performed by the following procedure: the single-stranded DNA is reacted with sulfite composition, if need under existence of scavenger, then hydrolyzed, and further, desulfonated under existence of alkali.

When a single-stranded DNA is provided to the above-described bisulfite reaction, usually it is provided as a solution which dissolves the single-stranded DNA, and said solution includes the one dissolved, for example, in Good's buffer solution such as MES and HEPES, phosphate buffer solution, Tris buffer solution, glycine buffer solution, borate buffer solution, sodium bicarbonate buffer solution, and sterile water, etc., having pH 6 to 8; and among them, the one dissolved in sterile water is preferable. The amount of single-stranded DNA in said solution is not limited specifically, but usually it is 10 to 100 ng in 1 to 10 µL of the solution.

The sulfites in the reaction of single-stranded DNA with sulfite composite in the above-described bisulfite reaction includes, for example, sodium bisulfite and ammonium sulfite, and, sodium bisulfite is preferable. The usage thereof is one so that the final concentration in the reaction solution gives 1 to 6 mol/L relative to usually 1 to 500 µL of a solution including 50 ng to 500 ng of single-stranded DNA. The scavenger described above includes, for example, hydroquinone compounds such as hydroquinone and the like. Said scavenger may be added so that the final concentration gives 1 to 5 mmol/L relative to 1 to 500 µL of a solution including 500 ng to 5 µg of single-stranded DNA. The reaction of said single-stranded DNA with sulfite is performed usually by making react at 30 to 70°C, preferably at 40 to 60°C, more preferably at 50 to 60°C, and usually for 40 minutes to 24 hours, preferably for 1 hour to 20 hours, more preferably for 4 hours to 16 hours.

The hydrolysis during the above-described bisulfite reaction is not limited specifically as long as it is a method usually performed in this field, and it is performed usually by heating at 30 to 70°C, preferably at 40 to 60°C, and usually for 40 minutes to 24 hours, preferably for 1 hour to 20 hours, more preferably for 4 hours to 16 hours. It should be noted that said hydrolysis treatment may be carried out simultaneously with the above-described reaction of single-stranded DNA with the sulfite.

It should be noted that, with respect to the single-stranded DNA which carried out the above-described hydrolysis, it is preferable to subject it to purification treatment before desulfonation treatment. Said purification treatment is the treatment to be carried out for removing high-concentration of sulfite salt to be used in the bisulfite reaction, and it may be carried out according to the method for purifying DNA to be carried out usually in this field. Specifically, there are included for example, a method in which chaotropic agent such as guanidine hydrochloride or sodium iodide is added to the single-stranded DNA or a solution including single-stranded DNA, and it is separated and purified by HPLC method etc.; for example, extraction and purification by a mixed solution of phenol/chloroform/isoamyl alcohol; alcohol precipitation method; purification by a column filled with silica gel; filtration method with filter, etc.; among them, the alcohol precipitation method is preferable. Said alcohol precipitation method is specifically performed as follows.

That is, to a 10 µL of solution including single-stranded DNA after hydrolysis, usually, 40 to 110 µL of alcohol and 30 to 100 µL of buffer solution are added, and centrifugal separation is carried out. After centrifugal separation, by removing supernatant and washing with alcohol, objective single-stranded DNA can be separated and purified. At the time when the above-described alcohol and buffer solution are added, to facilitate removal of supernatant after separation, 0.1 to 1 µL of Ethachinmate or glycogen may be added to 10 µL of the solution including single-stranded DNA. The above-described alcohol includes ethanol, isopropanol, and butanol, and the like; and, isopropanol is particularly preferable. In the bisulfite reaction pertaining to the present invention, although the reason is unclear, when isopropanol is used, only the objective single-stranded DNA can be precipitated efficiently and it will become possible to advance the reaction efficiently. The above described buffer solution includes, for example, Good's buffer solution such as MES and HEPES, phosphate buffer solution, Tris buffer solution, glycine buffer solution, borate buffer solution, and sodium bicarbonate buffer solution and so on; among them, Good's buffer solution such as MES and HEPES, Tris buffer solution, etc. are preferable, and Tris buffer solution is particularly preferable. The pH of these buffer solutions is usually 7 to 8, preferably 7 to 7.5, and concentration of buffer agent in the buffer solution is usually in the range of 0.1 to 5 mol/L, preferably 0.1 to 2 mol/L. The above-described centrifugal separation is not limited specifically as long as it is an aspect to be carried out usually in this field, and usually it is carried out by 10,000g to 22,000g for 10 to 30 minutes.

The desulfonation reaction in the above-described bisulfite reaction includes the same method as alkaline treatment in the section of the above-described single strand formation treatment, and a preferable aspect also includes the same one.

The reverse transcriptase reaction is usually employed in this field for synthesizing complementary strand DNA through the use of reverse transcriptase activity (reverse-transcription reaction activity: RNA-dependent DNA polymerase activity) which the reverse transcriptase has, and using single-stranded RNA as a template. However, in the present invention, the reverse transcriptase reaction is employed for synthesizing complementary strand DNA through the use of DNA-dependent DNA polymerase activity which the reverse transcriptase has, and using single-stranded DNA as a template. That is, the reverse transcriptase reaction in the present invention may be carried out according to the method for synthesizing complementary strand DNA by employing well known per se single-stranded RNA, which is usually used in this field, except for employing single-stranded DNA instead of single-stranded RNA as a template. The above-described single-stranded DNA, which carried out the bisulfite reaction pertaining to the present invention, (hereinafter, sometimes abbreviated as DNA after bisulfite reaction) may be subjected to the reverse transcriptase reaction, and may also be carried out using commercially available kit. Specifically, for example, to 1 µg quantity of nucleic acid of the DNA after bisulfite reaction, usually 50 to 500 Units, preferably 100 to 400 Units of reverse transcriptase, usually 0.1 to 1 µg, preferably 0.5 to 1 µg of primers for reverse transcriptase reaction, usually each 1 to 50 nmol, preferably 1 to 20 nmol of 4 kinds of deoxyribonucleotide triphosphate (dNTPs) are added, and reacted in a buffer solution (pH 7 to pH 8.5) such as Tris hydrochloric acid buffer solution, HEPES, and MOPS buffer solution, preferably a buffer solution (pH 8 to pH 8.5) such as Tris buffer solution and HEPES buffer solution, at usually 35 to 50°C, preferably at 40 to 50°C for usually 30 to 90 minutes, preferably for 30 to 60 minutes. Thereby, a single-stranded DNA, which is complementary to the DNA after bisulfite reaction, can be obtained. It should be noted that, in the case where the primer is random primer, it is necessary to advance annealing reaction before carrying out the above-described reaction. Therefore, when the reaction is carried out using random primer, after adding the above-described amount of reverse transcriptase, random primer, and 4 kinds of deoxyribonucleotide triphosphate (dNTPs) to the DNA after bisulfite reaction, annealing reaction is carried out usually at 20 to 40°C, preferably at 20 to 30°C for 5 to 30 minutes, preferably for 10 to 20 minutes, after that, the reverse transcriptase reaction is carried out at the above-described temperature for the above-described time.

It should be noted that, in the above-described reverse transcriptase reaction, it is preferable to stop the reaction by heating treatment or by adding reaction stop solution, after reaction. Said heat treatment is carried out usually at 65 to 100°C, preferably at 65 to 70°C for usually 15 to 60 minutes, preferably for 15 to 30 minutes. In addition, the reaction stop solution includes, for example, EDTA and the like, and its usage is one to provide final concentration to be usually 10 to 100 mmol/L, preferably 40 to 60 mmol/L.

In addition, in the above-described reverse transcriptase reaction, regents such as reducing agent of DTT (dithiothreitol) etc., potassium chloride, magnesium chloride, which are usually used at such reverse transcriptase reaction, may be added. Concentration and usage of these reagents may be selected appropriately from the range usually employed in this field.

The reverse transcriptase at the above-described reverse transcriptase reaction is not limited specifically as long as it has a DNA dependent DNA polymerase activity, which includes, for example, Moloney Murine Leukemia Virus (M-MLV) reverse transcriptase, Avian myeloblastosis virus (AMV) reverse transcriptase, and M-MLV reverse transcriptase (RNase H Minus);and, M-MLV reverse transcriptase (RNase H Minus) is preferable among them.

The primer for reverse transcriptase reaction may be the one which can anneal to DNA after bisulfite reaction, which becomes a template, and serves as a starting point of DNA chain extension. Number of nucleotides of the primer for reverse transcriptase reaction is usually 6 to 12 mer, preferably 6 to 10 mer, more preferably 6 to 9 mer. It should be noted that, when the single-stranded DNA pertaining to the present invention is unknown, it is preferable to use this random primer.

The above-described dNTPs is not limited specifically as long as it is a mixture of four kinds of deoxyribonucleotide triphosphate (dATP, dCTP, dGTP, dTTP) usually employed in this field.

A preferable example of the method for obtaining complementary DNA of uracilated DNA of the present invention will be explained below.
That is, for example, DNA is extracted from cell and the like using a DNA extraction kit and the like, then 1 µg of DNA is dissolved, for example, in sterile water to prepare a solution including DNA. When the obtained DNA is double-stranded DNA, to 3 to 5 µL of the solution including DNA, for example, 1 µL to 5 µL of 1 to 3 mol/L sodium hydroxide etc. is added, and reacted at 30 to 50°C for 10 to 30 minutes to make the DNA single strand.

After that, to 4 to 8 µL of a solution including the obtained single-stranded DNA, 30 to 50 µL of 2 to 3 mol/L sodium bisulfite, and if need, 3 to 5 µL of 20 to 50 mmol/L hydroquinone are added, and heated at 80 to 90°C for 60 to 100 minutes. By reacting under said condition, cytosine in the single-stranded DNA is sulfonated, and at the same time, the sulfonated cytosine can be hydrolyzed. Subsequently, 5 to 10 times larger volume than the post-hydrolysis solution of 1 mol/L Tris buffer solution (pH 7.5 to 8.0) and isopropanol are added by a ratio of 40:60 to 60:40, preferably by a ratio of 40:60 to 50:50, respectively, to precipitate the single-stranded DNA after hydrolysis. On this occasion, when 1 to 3 µL of Ethachinmate is added, confirmation of DNA precipitation will become easy. After that, centrifugal separation is carried out by 10,000 to 20,000g for 10 to 20 minutes and then removes the supernatant, and the obtained DNA is washed with ethanol. Thereby, the single-stranded DNA after hydrolysis can be extracted and purified. Furthermore, 1 µg of the obtained single-stranded DNA is dissolved, for example, in 30 to 40 µL of sterile water, and to said solution, 5 to 20 µL of 1 to 3 mol/L sodium hydroxide is added and reacted at 30 to 40°C for 20 to 60 minutes to desulfonate. After that, if need, the purification is performed by removing low molecular weight DNA using a commercially available kit, etc. Thereby, the bisulfite reaction is completed, and single-stranded DNA, in which non-methylated cytosine has been uracilated efficiently, can be obtained.

Next, to 5 to 10 µL of a solution containing 1 µg of single-stranded DNA obtained by bisulfite reaction, for example, 1 to 2 µL of 1 to 3 µg/µL random primers (5 to 15 mer), 1 to 2 µL of 100 to 400 Units reverse transcriptase, and 5 to 10 µL of mixed solution of 1 to 3 mmol/L of 4 kinds of deoxyribonucleotide triphosphate (dNTPs) are added; and annealing reaction is performed usually at 20 to 40°C for 10 to 20 minutes; after that, by carrying out extension reaction with reacting at 37 to 50°C for usually 30 to 60 minutes, reverse transcriptase reaction is carried out. After the reaction, the obtained single-stranded DNA is purified by removing low molecular weight DNA using, for example, commercially available kit, etc.
By the above-described procedure, the DNA, which is complementary to single-stranded DNA in which non-methylated cytosine has been uracilated (hereinafter, sometimes abbreviated as a complementary DNA of uracilated DNA) can be obtained.

### [A method for amplifying DNA which is complementary to single-stranded DNA in which non-methylated cytosine has been uracilated (a method for amplifying complementary DNA of uracilated DNA of the present invention)]

The method for amplifying complementary DNA of uracilated DNA of the present invention can amplify efficiently the DNA, which is complementary to the above-described single-stranded DNA in which non-methylated cytosine has been uracilated, by subjecting single-stranded DNA to 1) bisulfite reaction, 2) reverse transcriptase reaction and 3) PCR reaction in this order.

As for the above-described bisulfite reaction and reverse transcriptase reaction, the same method as described in a section of the method for obtaining complementary DNA of uracilated DNA of the present invention is included, and the preferable condition, etc. are also the same.

The PCR reaction in the method for amplifying complementary DNA of uracilated DNA of the present invention may be carried out according to the method well known per se, for example, the method described in Nucleic Acids Research, 1991, Vol. 19, 3749, BioTechniques, 1994, Vol.16, 1134-1137, and specifically, is carried out as follows. That is, to 1 to 100 ng of quantity of nucleic acid of single-stranded DNA (herein after, sometimes abbreviated as post-reverse-transcriptase reaction DNA), which becomes a template, obtained by the above-described reverse transcriptase reaction, usually 0.1 to 100 pmol, preferably 0.1 to 50 pmol of 2 kinds of primers, respectively, usually 1 to 10 Units, preferably 2.5 to 5 Units of DNA polymerase, and usually 0.01 to 20 µmol, preferably 0.01 to 10 µmol of a mixture of 4 kinds of deoxyribonucleotide triphosphate (dNTPs) are added. By setting, for example, the processes of (1) 93 to 98°C for 1 to 10 minutes → (2) 93 to 98°C for 10 to 30 seconds → (3) 50 to 60°C for 10 to 30 seconds → (4) 68 to 72°C for 30 seconds to 5 minutes as 1 cycle, and by carrying out for 20 to 40 cycles in a buffer solution such as Tricine buffer solution and Tris buffer solution, etc., the DNA complementary to the DNA after bisulfite reaction can be amplified and obtained. In the above-described PCR reaction, after the reaction, it is preferable to purify the obtained DNA by purification method to be used usually in this field, such as, for example, extraction by a mixed solution of phenol/chloroform/isoamyl alcohol, alcohol precipitation, column purification, filtration by a filter, etc. In addition, after the above-described purification, it is more preferable to extract DNA having objective base pair (bp). Said extraction method includes the method well known per se, for example, the method using agarose gel electrophoresis, the method using liquid chromatography, and the method using electrophoresis on polyacrylamide gel and the like as described in Labo Manual for Genetic Engineering, Expanded Edition. In addition, the double-stranded DNA pertaining to the present invention which is obtained by the above-described PCR reaction may be subjected to further PCR reaction to obtain more amount of objective DNA.

Two kinds of primers in the above-described PCR reaction is the one which includes a part of DNA after reverse-transcriptase reaction and the one which includes a part of complementary strand of DNA after reverse-transcriptase reaction, which is a template, and number of nucleotides thereof is usually 12 to 40, preferably 15 to 35, more preferably 18 to 30.

The DNA polymerase in the above-described PCR reaction may be any DNA polymerase as long as it is usually used in this field, and, the one which has 3'→5' exonuclease activity is preferable. Specifically, α-type DNA polymerase such as, for example, Pfu DNA polymerase, KOD DNA polymerase is preferable, among them, the KOD DNA polymerase is particularly preferable. By using such DNA polymerase having 3'→5' exonuclease activity, it becomes possible to amplify accurately the DNA complementary to the DNA after bisulfite reaction, as a consequence, highly accurate detection of methylated cytosine becomes possible.

The above-described dNTPs is not limited especially if it is a mixture of 4 kinds of deoxyribonucleotide triphosphate (dATP, dCTP, dGTP, dTTP) usually employed in this field.

A preferable example of the method for amplifying complementary DNA of uracilated DNA of the present invention is explained below.

That is, first, as described in the section of the method for obtaining complementary DNA of uracilated DNA of the present invention, by subjecting a single-stranded DNA to bisulfite reaction and reverse transcriptase reaction in this order, the complementary DNA of uracilated DNA is obtained. After that, the obtained DNA is subjected to the PCR reaction. That is, to 1 to 3 µL of a solution including 100 ng to 1 µg of single-stranded DNA obtained by the reverse transcriptase reaction, 5 to 10 µL of 1 to 10 µmol/L primer for upstream of the DNA of amplification target and 5 to 10 µL of 1 to 10 µmol/L primer for downstream of the DNA of amplification target, 5 to 10 µL of a mixture of 4 kinds of 1 to 5 mmol/L deoxyribonucleotide triphosphate (dNTPs), and 20 to 30 µL of 1 to 5 Units KOD DNA polymerase are added. Next, for example, by setting the reactions at 93 to 98°C for 1 to 10 minutes → 93 to 98°C for 10 to 30 seconds → 50 to 60°C for 10 to 30 seconds → 68 to 72°C for 30 seconds to 5 minutes as 1 cycle, 20 to 40 cycles of reaction are carried out. Thereby, the DNA complementary to a DNA, in which non-methylated cytosine has been uracilated, is amplified. After that, said double-stranded DNA is subjected to electrophoresis with using, for example, agarose gel or nondenaturing polyacrylamide gel, and DNA of desired chain length is extracted. It should be noted that, if need, the obtained DNA may be purified for example, by extraction with a mixed solution of phenol/chloroform/isoamyl alcohol. By the above procedure, the complementary DNA of uracilated DNA can be amplified.

### [A method for detecting methylated cytosine of the present invention]

The method for detecting methylated cytosine of the present invention is performed by subjecting single-stranded DNA to 1) bisulfite reaction, 2) reverse transcriptase reaction and 3) PCR reaction in this order, and by carrying out nucleotide sequence analysis of the obtained PCR amplification product

In the method for detecting methylated cytosine of the present invention, bisulfite reaction, reverse transcriptase reaction, and PCR reaction are the same methods as described in a section of the method for obtaining complementary DNA of uracilated DNA of the present invention and the method for amplifying complementary DNA of uracilated DNA of the present invention, and the preferable condition, etc. are also the same.

As the above-described nucleotide sequence analysis, there is no particular limitation as long as it is a method for analyzing nucleotide sequence usually employed in this field. For example, it may be carried out according to a routine procedure such as a fluorescent dye terminator sequencing method and Sanger's method which are described in Lab Manual for Genetic Engineering, and Handbook of Gene Technology, etc. Specifically, for example, a complementary DNA of uracilatd DNA, which is obtained by the amplification method of the present invention, is incorporated in a vector; and the obtained recombinant vector is transfected into competent cell; said competent cell is cultured; and from there, a plasmid including complementary DNA of uracilated DNA is extracted; and using said plasmid, decoding is performed by, for example, a sequencer and the like. By comparing thus obtained nucleotide sequence with the nucleotide sequence of normal DNA which has not been subjected to the bisulfite reaction, methylated cytosine can be detected. That is, in the bisulfite reaction pertaining to the present invention, since all cytosine except for methylated cytosine are converted into uracil, the methylated cytosine can be detected by finding out cytosine not converted into uracil in the obtained nucleotide sequence.

The method for incorporating the above-described complementary DNA of uracilated DNA into a vector includes, specifically, for example, a method for inserting complementary DNA of uracilated DNA into a vector using T4 DNA ligase after the vector such as plasmid, cosmid, and phagemid, and complementary DNA of uracilated DNA are blunt-ended by T4 DNA polymerase and the like, or a TA cloning method, in which adenine (A) is added to the complementary DNA of uracilated DNA, and said adenine-added complementary DNA of uracilated DNA is incorporated into a thymine base- added vector, using T4 DNA ligase. Among them, the TA cloning method is preferable because it does not require cleaving both DNA to be inserted and vector by restriction enzyme, and it is simple.

Said TA cloning method is carried out, specifically, for example as follows. That is, to 100 ng of the complementary DNA of uracilated DNA after PCR reaction, 1 to 5 Units of *Taq* DNA polymerase is added, and reacted at 55 to 75°C for 10 to 30 minutes, and adenine is added to 3'-terminal of the complementary DNA of uracilated DNA. It should be noted that, on the occasion of said reaction, 0.01 to 20 nmol of dATP relative to 1 µg of the complementary DNA of uracilated DNA may be added to the reaction solution, however, by adding *Taq* DNA polymerase to the PCR reaction solution, this addition step can be skipped. In addition, with respect to the complementary DNA of the adenine-added uracilated DNA, after synthetic reaction, it is preferable to purify the obtained DNA by a method such as extraction with a mixed solution of phenol/chloroform/isoamyl alcohol, alcohol precipitation, column purification, and filtration by a filter, etc. Subsequently, to 10 to 100 ng of complementary DNA of the adenine-added uracilated DNA, thymine base-added vector for *E. coli* transformation and 300 to 3000 Units of T4 DNA ligase are added, and by reacting at 10 to 40°C for 30 to 90 minutes, a recombinant vector, in which the complementary DNA of uracilated DNA is incorporated, can be obtained.

A method for transforming the above-described recombinant vector to competent cell includes, for example, a heat shock method by heating at 35 to 45°C for 20 to 90 minutes, and an electroporation method in which 1.5 to 2.5 kV of electric pulse is applied, but the electroporation method is more preferable. As the competent cell to be used herein, if it is *E. coli* or *B. subtilis,* which is usually used, any one of them can be employed, and its usage to be used may be set appropriately within the range usually employed.

Cultivation of the above-described competent cell is performed, for example, on a medium such as LB agar medium including 30 to 150 µg/mL of ampicillin, or M9 agar medium including 30 to 150 µg/mL of ampicillin, at 30 to 40°C for 12 to 20 hours. It should be noted that, as the above-described medium, either one of natural medium or synthetic medium etc. may be employed as long as it contains carbon source, nitrogen source and inorganic salts, which become nutritional source of bacteria, and yeast extract as a growth factor, and it enables to culture the transformant efficiently. Said carbon source includes carbohydrates such as glucose, fructose, sucrose and starch; organic acids such as acetic acid and propionic acid, and alcohols such as ethanol and propanol. Said nitrogen source includes ammonia; ammonium salt of inorganic acid or organic acid such as ammonium chloride, ammonium sulfate, ammonium acetate, and ammonium phosphate; or other nitrogen-containing compounds, in addition, peptone, tryptone, meat extract, and Corn Steep Liquor, and the like. The inorganic salts include monobasic potassium phosphate, dibasic potassium phosphate, magnesium phosphate, magnesium sulfate, sodium chloride, ferrous sulfate, manganese sulfate, copper sulfate, and calcium carbonate, etc.

As a method for extracting the plasmid including the complementary DNA of uracilated DNA from cultured competent cell, for example, first, the DNA derived from plasmid, which includes the complementary DNA of uracilated DNA in a colony is amplified by colony PCR method. After that, may be carried out whether the objective plasmid is amplified in a colony is determined, for example, by electrophoresis, and from the colony, which is identified insertion of objective plasmid, the objective plasmid is extracted.

Said colony PCR method is carried out, for example, as follows. That is, to the cultured colony, usually 0.1 to 100 pmol, preferably 0.1 to 50 pmol of 2 kinds of PCR primers for detection of objective nucleotide sequence, respectively, usually 0.01 to 20 nmol, preferably 0.01 to 10 nmol of 4 kinds of mixed deoxyribonucleotide triphosphate (dNTPs), and usually 1 to 10 Units, preferably 1 to 5 Units of DNA polymerase are added, and in a buffer solution such as Tricine buffer solution, Tris hydrochloric acid buffer solution and the like, and for example, by setting the reactions (1) at 93 to 98°C for 1 to 10 minutes → (2) at 93 to 98°C for 10 to 30 seconds → (3) at 50 to 60°C for 10 to 30 seconds → (4) at 68 to 72°C for 30 seconds to 5 minutes as 1 cycle, 25 to 40 cycles of reaction are carried out. The above-described 2 kinds of primer include the one which is designed to be able to amplify the objective DNA. That is, the one which includes the entire or a part of uracilated DNA and the one which includes the entire or a part of the complementary DNA of uracilated DNA, or the sequences derived from vector which locate at both end of the inserted complementary DNA of uracilated DNA, and, the sequences derived from vector, which locate in the both end of the complementary DNA of uracilated DNA, are preferable. That is, in the method of the present invention, since non-methylated cytosine is uracilated, and said uracil is read as thymine at the time of reverse transcriptase reaction, if all cytosine were non-methylated cytosine, the complementary DNA of uracilated DNA would be consisted of 3 nucleotides. Therefore, in order to carry out the PCR reaction efficiently, the sequence of the vector origin located in the both ends of the complementary DNA of uracilated DNA, which can be constituted by 4 nucleotides, is preferable as the primer. The number of nucleotides of the above-described primer is usually 12 to 30, preferably 15 to 25, more preferably 18 to 22. The above-described DNA polymerase may be any DNA polymerase as long as it is usually employed in this field, and specifically, for example, there are included *Taq* DNA polymerase, *Tth* DNA polymerase, and KOD DNA polymerase, etc., and among them, *Taq* DNA polymerase and KOD DNA polymerase, etc. are preferable.

The electrophoresis method to be carried out after the above-described colony PCR reaction may be any electrophoresis method, which is usually employed in this field, as long as it can determine the number of nucleotides from the mobility; however agarose gel electrophoresis method is preferable. It should be noted that, the electrophoretic condition in said electrophoresis method may be set appropriately according to well-known method.

As the method for taking out plasmid from the above-described colony part, plasmid may be extracted by well-known plasmid extraction method such as alkaline SDS method, after shaking culture, for example, described in Lab Manual for Genetic Engineering (Maruzen Co., Ltd.), and Handbook of Gene Technology (Yodosha Co., Ltd.). It should be noted that, extraction of plasmid may be carried out using commercially available kit. As the culture medium in the above-described shaking culture, the same medium as described in the section of cultivation of competent cell can be used except for making solution without using agarose, and preferable cultivation time and cultivation temperature are also the same range as described in the section of cultivation of competent cell.

Preferable example of the method for detecting methylated cytosine of the present invention is explained below.

That is, first, as descried in the sections of the method for obtaining complementary DNA of uracilated DNA of the present invention and the method for amplifying complementary DNA of uracilated DNA of the present invention, by subjecting single-stranded DNA to bisulfite reaction, reverse transcriptase reaction, and PCR reaction in this order, the complementary DNA of uracilated DNA is obtained. To 5 µL to 10 µL of a solution including 100 ng to 1 µg of said complementary DNA of uracilated DNA, 0.5 to 1 µL of 1 to 5 Units of *Taq* DNA polymerase are added, and reacted at 55 to 75°C for 10 to 30 minutes to add adenine to 3'-terminal of the complementary DNA of uracilated DNA. It should be noted that, with respect to the adenine-added complementary DNA of uracilated DNA, after synthetic reaction, it is preferable subject it to purification process. Next, to 1 to 5 µL of sterile water including 10 to 100 ng of the adenine-added complementary DNA of uracilated DNA, 1 to 3 µL of 10 to 100 ng of a thymine-added vector for E. *coli* transformation, and 1 to 3 µL of 300 to 3000 Units of T4 DNA ligase are added, and reacted at 10 to 20°C for 30 to 240 minutes to obtain a recombinant vector incorporated with the complementary DNA of uracilated DNA. After that, 10 to 100 ng of the obtained recombinant vector is added to 10⁸ to 10⁹ cells of competent cell, and transformation is performed by applying 1.5 to 2.5 kV of electric pulse. Furthermore, cells are cultured, for example, on agar medium including 30 to 150 µg/mL of ampicillin, 1% (w/v) of tryptone, 0.5% (w/v) of yeast extract, and 1% (w/v) of sodium chloride at 30°C to 40°C for 12 hours to 20 hours. Next, the obtained culture is subjected to the colony PCR. Specifically, to 1 µL to 10 µL of sterile water dissolved colony, 1 to 10 µmol/L of 2 kinds of primers, which are designed to be able to amplify the objective DNA, respectively, usually 1 to 5 µL of 1 to 10 mmol/L of 4 kinds of mixed deoxyribonucleotide triphosphate (dNTPs) and 0.5 to 1 µL of 1 to 5 Units Taq DNA polymerase are added, and reacted in a buffer solution such as Tricine buffer solution and Tris hydrochloric acid buffer solution, and by setting the reaction (1) at 93 to 98°C for 1 to 10 minutes → (2) at 93 to 98°C for 10 to 30 seconds → (3) at 50 to 60°C for 10 to 30 seconds → (4) at 68 to 72°C for 30 seconds to 5 minutes as 1 cycle, 25 cycles to 40 cycles of reaction are carried out. After that, existence of the objective DNA in the colony is determined by agarose gel electrophoresis, and identified colonies are collected. After carrying out shaking culture of the collected colonies in LB medium, the objective DNA is taken out from the culture medium using, for example, commercially available plasmid extraction kit and the like, and nucleotide sequence of the DNA is decoded by a sequencer, etc. Comparing the obtained sequence with the normal nucleotide sequence which is not subjected to the bisulfite reaction, and by finding out the cytosine which is not uracilated in the decoded nucleotide sequence, the methylated cytosine can be detected.

Hereinafter, the present invention will be explained in more detail by referring to Experimental Example, Examples, and Comparative Examples and so on, however, the present invention is not limited thereto in any way.

### Example

### Experimental Example 1: Extraction of mouse genomic DNA

Using QuickGene SP kit DNA tissue (produced by Fuji Film Co., Ltd.), from 1 x 10⁶ cells of mouse embryonic stem cell (ES cell) and mouse embryonic fiblobrast cell (MEF cell), genomic DNA was extracted according to an attached instruction manual.

Then, confirmation of presence of ES cell-derived *Nanog* gene and *Rex1* gene, and MEF cell-derived *Nanog* gene and *Rex1* gene was carried out.

That is, sterile water was added to each 10 µg of the obtained purified mouse genomic DNA derived from ES cell and MEF cell so as to give the total volume 33.5 µL. Two kinds of these solutions were prepared, and the PCR amplification was carried out for promoter region of mouse *Nanog* gene and *Rex1* gene. That is, to the above-described sterile water, 5 µL of 10 × Gene *Taq* Universal Buffer (produced by Nippon Gene Co. Ltd.), 5 µL of dNTPs mixed solution (2.5 mmol/each) (produced by Nippon Gene Co. Ltd.), and 0.5 µL of Gene *Taq* NT (produced by Nippon Gene Co. Ltd.) were added, and to this solution, 3 µL of 5 µmol/L of PCR primer Forward solution and Reverse solution for *Nanog* gene [Forward: 5' CTGTGAATTCACAGGGCTGGTGGG 3' (Nucleotide sequence 1), Reverse: 5'CAACCAAATCAGCCTATCTGAAGGCC 3' (Nucleotide sequence 2)], respectively, or 3 µL of 5 µmol/L PCR primer Forward solution and Reverse solution for *Rex1* gene [Forward: 5' GGGTCACCTGAAGGGCCAGGGGCC 3' (Nucleotide sequence 3), Reverse: 5' CTTGGACCCCTCCCTTTTTAGATGG 3' (Nucleotide sequence 4)], respectively, were added to make the total volume 50 µL, and by setting the reactions at 95°C for 2 minutes, at 95°C for 20 seconds, and at 68°C for 30 seconds as 1 cycle, 30 cycles of PCR reaction were carried out. After that, the obtained respective PCR amplified products were electrophoresed using 1.5% agarose gel, and the presence of ES cell derived *Nanog* gene and *Rex1* gene, and MEF cell derived *Nanog* gene and *Rex1* gene were confirmed.

The result of *Nanog* gene was shown in Fig. 1, and the result of *Rex1* gene was shown in Fig. 2. From said results, the presence of *Nanog* gene and *Rex1* gene could be confirmed in both ES cell and MEF cell.

### Example 1: Uracilation of non-methylated cytosine in the ES cell-derived Nanog gene, ES cell-derived Rex1 gene, and ES cell-derived CD133 gene by the method of the present invention

### (1) Single strand formation of genomic DNA by alkaline treatment

One µg of ES cell-derived genomic DNA obtained in Example 1 was placed in a tube, and 3 tubes were prepared as a tube for *Nanog* gene, a tube for *Rex1* gene, and a tube for *CD133* gene. After each of these was adjusted to 4.5 µL by adding sterile water, 2 µL of 1 mol/L sodium hydroxide (produced by Wako Pure Chemical Industries Co., Ltd.) was added to make the total volume 6.5 µL, and incubated at 37°C for 20 minutes to make each genomic DNA single stranded.

### (2) Bisulfite reaction

To 6.5 µL of the solution including single-stranded genomic DNA in each three tubes, 3.5 µL of 30 mmol/L hydroquinone (produced by Wako Pure Chemical Industries Co., Ltd.), 40 µL of 2.5 mol/L sodiumhydrogensulfite (produced by Wako Pure Chemical Industries Co., Ltd.) were added to make the total volume 50 µL, and incubated at 55°C for 16 hours. Then, 350 µL of 1 mol/L Tris buffer solution (pH 7.5) (produced by Nippon Gene Co., Ltd.), 1 µL of Ethachinmate (produced by Nippon Gene Co., Ltd.), and 400 µL of isopropanol (produced by Wako Pure Chemical Industries Co., Ltd.) were added, respectively, and centrifugal separation was carried out by 18800 x g for 10 minutes. After that, the supernatant was removed and washed with 75% ethanol (produced by Wako Pure Chemical Industries Co., Ltd.), and then, sterile water was added to make the total volume 35 µL.
Next, to 35 µL of the solution containing genomic DNA in each three tubes, 15 µL of 1 mol/L sodium hydroxide (produced by Wako Pure Chemical Industries Co., Ltd.) was added to make the total volume 50 µL, and incubated at 37°C for 20 minutes, and thus the desulfonation reaction was performed. Then, 12 µL of 10 mol/L ammonium acetate (produced by Nippon Gene Co., Ltd.) and 125 µL of ethanol (produced by Wako Pure Chemical Industries Co., Ltd.) were added, respectively, and centrifugal separation was carried out by 18800 x g for 10 minutes. Subsequently, the supernatant was removed and washed with 75% ethanol (produced by Wako Pure Chemical Industries Co., Ltd.), and then, sterile water was added so as to adjust the total volume 50 µL. After that, using QIAquick PCR Purification Kit (QIAGEN, Inc.), low molecular weight DNA was removed according to an attached instruction manual. Furthermore, to the solution after removal procedure, 1 µL of Ethachinmate (produced by Nippon Gene Co., Ltd.), 5 µL of 3 mol/L sodium acetate (produced by Nippon Gene Co., Ltd.), and 125 µL of ethanol (produced by Wako Pure Chemical Industries Co., Ltd.) were added, respectively, and centrifugal separation was carried out by 18800 x g for 10 minutes. Finally, the supernatant was removed and washed with 75% ethanol (produced by Wako Pure Chemical Industries Co., Ltd.), and then sterile water was added so as to make the total volume 6 µL.

### (3) Reverse transcriptase reaction

To each 6 µL of solution containing genomic DNA after bisulfite reaction in the 3 tubes, 1 µL of random primer (6 mer, 1 µg/µL) (produced by Takara Bio Inc.) was added to make the total volume 7 µL; and after the solution was incubated at 90°C for 1 minute, and cooled down immediately with ice, and thus heat denaturation was performed. After that, 4 µL of 5 x buffer (produced by TOYOBO Co. Ltd.), 8 µL of 2.5 mmol/L dNTPs mixed solution (produced by Nippon Gene Co. Ltd.), and 1 µL of ReverTra Ace (produced by TOYOBO Co. Ltd.) were added, respectively, to make the total volume 20 µL, and after incubation at 25°C for 10 minutes, by performing incubation at 42°C for 30 minutes, elongation reaction by reverse transcriptase was carried out. Subsequently, using QIAquick PCR Purification Kit (QIAGEN, Inc.), low molecular weight DNA was removed according to an attached instruction manual. After that, to the solution after removal treatment of low molecular weight DNA, 1 µL of Ethachinmate (produced by Nippon Gene Co., Ltd.), 5 µL of 3 mol/L sodium acetate (produced by Nippon Gene Co., Ltd.), and 125 µL of ethanol (produced by Wako Pure Chemical Industries Co., Ltd.) were added, respectively, and centrifugal separation was carried out by 18800 x g for 10 minutes. Further, the supernatant was removed and washed with 75% ethanol (produced by Wako Pure Chemical Industries Co., Ltd.), and then sterile water was added so as to make the total volume 10 µL.

### (4) PCR reaction

To each 1 µL of a solution containing genomic DNA in 3 tubes which was performed reverse transcriptase reaction, 7 µL of sterile water, 25 µL of 2 x PCR buffer for KOD FX (produced by TOYOBO Co. Ltd.), 10 µL of 2.5 mmol/L dNTPs mixed solution (produced by TOYOBO Co. Ltd.), and 1 µL of KOD FX (produced by TOYOBO Co. Ltd.) were added, and to this solution each 3 µL of 5 µmol/L PCR primer Forward solution and Reverse solution for *Nanog* gene [Forward: 5' TTGTGAATTTATAGGGTTGGTGGG 3' (Nucleotide sequence 5), Reverse: 5' CAACCAAATCAACCTATCTAAAAACC 3' (Nucleotide sequence 6)], each 3 µL of 5 µmol/L PCR primer Forward solution and Reverse solution for *Rex1* gene [Forward: 5' GGGTTATTTGAAGGGTTAGGGGTT 3' (Nucleotide sequence 7), Reverse: 5' CTTAAACCCCTCCCTTTTTAAATAA 3'(Nucleotide sequence 8)], or each 3 µL of 5 µmol/L PCR primer Forward solution and Reverse solution for *CD133* gene [Forward: 5' GTTTTTTAAATTATTGAGTTTTGTGGAG 3' (Nucleotide sequence 9), Reverse: 5' CACCACAAAAATAATTAAATAAAAACCC 3' (Nucleotide sequence 10)] were added to make the total volume 50 µL, and by setting the reaction at 94°C for 2 minutes → at 98°C for 10 seconds → at 55°C for 20 seconds → at 68°C for 30 seconds as 1 cycle, 35 cycles of PCR reaction were carried out. After that, the obtained respective PCR amplified products were fractionated by electrophoresis using 1.5% agarose gel, and whether the objective DNA was amplified was examined.

The result of the electrophoresis of *Nanog* gene derived from ES cell was shown in Fig. 1; the result of the electrophoresis of *Rex1* gene derived from ES cell was shown in Fig. 2; and the result of the electrophoresis of *CD133* gene derived from ES cell was shown in Fig. 3, respectively.

### (5) Cloning and nucleotide sequence analysis of PCR amplification product

To each 9 µL of 3 kinds of solutions after PCR reaction, which were confirmed amplification by electrophoresis, 1 µL of 10 x A-attachment Mix (produced by TOYOBO Co. Ltd.) was added to make the total volume 10 µL, and incubated at 60°C for 30 minutes to add adenine to 3'-terminal of the PCR amplified products. Next, using QIAquick PCR Purification Kit (QIAGEN, Inc.), low molecular weight DNA was removed according to an attached instruction manual. After that, to the solution after removal procedure of low molecular weight DNA, 1 µL of Ethachinmate (produced by Nippon Gene Co., Ltd.), 5 µL of 3 mol/L sodium acetate (produced by Nippon Gene Co., Ltd.), and 125 µL of ethanol (produced by Wako Pure Chemical Industries Co., Ltd.) were added, respectively, and centrifugal separation was carried out by 18800 x g for 10 minutes. Further, the supernatant was removed and washed with 75% ethanol (produced by Wako Pure Chemical Industries Co., Ltd.), and then sterile water was added so as to make the total volume 3 µL.

To each 3 µL of 3 kinds of solutions which comprise adenine-added PCR amplified products, 1 µL of pGEM-T Easy Vector (produced by Promega Corporation) and 4 µL of DNA Ligation Kit (produced by Takara Bio Inc.) were added to make the total volume 8 µL, and the PCR amplification product was inserted in the vector by incubating this solution at 16°C for 60 minutes. Next, using QIAquick PCR Purification Kit (QIAGEN, Inc.), purification of the vector was carried out. After that, to the solution after purification treatment, 1 µL of Ethachinmate (produced by Nippon Gene Co., Ltd.), 5 µL of 3 mol/L sodium acetate (produced by Nippon Gene Co., Ltd.), and 125 µL of ethanol (produced by Wako Pure Chemical Industries Co., Ltd.) were added, respectively, and centrifugal separation was carried out by 18800 x g for 10 minutes. Further, the supernatant was removed and washed with 75% ethanol (produced by Wako Pure Chemical Industries Co., Ltd.), and then sterile water was added so as to make the total volume 2 µL.

To each 1 µL of 3 kinds of solutions which comprise the PCR amplified products inserted in a vector, a 40 µL of E. *coli* (XL10 Gold, produced by Stratagene Corporation, 10⁹ cells) was added, and transformation was carried out by the electroporation method. Next, the transformed E. *coli* cells were cultured in LB agar medium at 37°C overnight. From colonies of cultured E. *coli,* a portion was picked up and dissolved in 5.9 µL of sterile water, and to said sterile water, 1 µL of 10 x Gene *Taq* Universal Buffer (produced by Nippon Gene Co., Ltd.), 1 µL of dNTPs mixed solution (each 2.5 mmol) (produced by Nippon Gene Co., Ltd.), 0.1 µL of Gene *Taq* NT (produced by Nippon Gene Co., Ltd.), and each 1 µL of 2 kinds of 5 µmol/L primers having sequence derived from pGEM-T Easy Vector [5' CCAGTCACGACGTTGTAAAACG 3' (Nucleotide sequence 11) and 5' CACACAGGAAACAGCTATGACC 3' (Nucleotide sequence 12), designed to give chain length of inserted fragment + 250 bp] were added, respectively, to make the total volume 10 µL, and by setting the reactions at 95°C for 2 minutes, at 95°C for 20 seconds, at 60°C for 20 seconds, at 72°C for 30 seconds as 1 cycle, 30 cycles of colony PCR reaction was carried out. After that, the obtained respective colony PCR amplified products were fractionated by electrophoresis using 1.5% agarose gel, and whether the objective DNA was amplified was examined. By employing the colony which was confirmed insertion of the vector, shaking culture was carried out in LB medium at 37°C overnight. After that, using the cultured broth, plasmid was extracted with the use of QuickGene Plasmid kit SII (produced by Fujifilm Corporation).

As for the 3 kinds of the obtained plasmids, decoding of nucleotide sequence was carried out by Applied Biosystems 3730x1 DNA Analyzer (Life Technologies Japan Ltd.), with the use of primer having sequence [5' CACACAGGAAACAGCTATGACC 3' (Nucleotide sequence 12)] derived from pGEM-T Easy Vector (produced by Promega Corporation).

The result of decoding of the *Nanog* gene was shown in Fig. 4; the result of decoding of the *Rex1* gene was shown in Fig. 5; the result of decoding of the *CD133* gene was shown in Fig. 6, respectively.

In Example 1, using genomic DNA extracted from ES cell, uracilation of non-methylated cytosine in the DNA and amplification of the DNA using the uracilated DNA as a template were attempted according to the method of the present invention. Specifically, non-methylated cytosine in DNA within each promoter region of *Nanog* gene, *Rex1* gene, and *CD133* gene which were stem cell marker was uracilated, and amplification of DNA using the DNA after uracilation as a template was carried out. In consequence, from the results of Fig. 1 to Fig. 3, it turned out that the respective genes in the ES cell were amplified by the method of the present invention.

Since the DNA after bisulfite reaction would become high thymine content, if high adenine content primer is used, nonspecific amplified products is easy to be formed, it may be difficult to acquire the objective amplification product. Practically, in the Comparative Examples 1 and 2 by the conventional method shown below, sufficient quantity of the PCR amplification product has not been acquired. On the other hand, in the method of the present invention, as is clear from Fig. 1 to Fig. 3, sufficient quantity of the PCR amplification product has been acquired. Especially, in the PCR amplification of the promoter region of *CD133,* PCR primer of 60.7% adenine content was used, and although it was PCR amplification in strict conditions, sufficient quantity of the PCR amplification product has been acquired. In the method of the present invention, the reverse transcriptase reaction and the PCR amplification reaction by α-type DNA polymerase were carried out after bisulfite reaction, and it is conceivable that the PCR amplification efficiency was improved as a result of performing these reactions continuously. In particular, since α-type DNA polymerase was used as a DNA polymerase for PCR amplification, it is conceivable that the DNA after bisulfite reaction has been amplified with sufficient accuracy by the proofreading activity. Consequently, from the results of Fig. 1 to Fig. 3, it turns out that the PCR amplification efficiency by the method of the present invention is good, and the acceptable range is wider.

In addition, as is clear from the results of Fig. 4 to Fig. 6, it turned out that any cytosine in *Nanog* gene, *Rex1* gene, and *CD133* gene was converted into uracil (on the result of decoding, uracil is written as thymine). Since the cytosine of promoter region of ES cell, which is a stem cell, is not usually methylated, from said result, it was shown that, according to the method of the present invention, all cytosine had been converted into uracil, and cytosine could be converted into uracil with high precision.

### Example 2: Uracilation of non-methylated cytosine in the MEF cell-derived Nanog gene, MEF cell-derived Rex1 gene, and MEF cell-derived CD133 gene according to the method of the present invention

Experiment was carried out by the same way as carried out in Example 1 (1) to (4) except for using MEF cell-derived genomic DNA obtained in Experimental Example 1 as a genomic DNA, instead of using ES cell-derived genomic DNA obtained in Experimental Example 1. That is, using MEF cell-derived genomic DNA, and according to the method described in Example 1, alkaline treatment, bisulfite reaction, reverse transcriptase reaction, and the PCR reaction were carried out, and said amplification product of the PCR was fractionated by electrophoresis using 1.5% agarose gel, and whether the objective DNA was amplified was confirmed.

The electrophoretic result of *Nanog* gene was shown in Fig. 1; the electrophoretic result of *Rex1* gene was shown in Fig. 2; and the electrophoretic result of *CD133* gene was shown in Fig. 3, respectively.

Each amplification product of the above-described PCR reaction which was identified by electrophoresis was subjected to ligation reaction with pGEM-T Easy Vector (produced by Promega Corporation) by the same manner as described in Example 1 (5), and after transformation of *E. coli,* plasmid was collected and the nucleotide sequence was decoded. The decoded result of MEF cell-derived *Nanog* gene was shown in Fig. 4, the decoded result of MEF cell-derived *Rex1* gene was shown in Fig. 5, and the decoded result of MEF cell-derived *CD133* gene was shown in Fig. 6, respectively.

In Example 2, using genomic DNA extracted from MEF cell, uracilation of non-methylated cytosine in DNA and amplification of the DNA using the uracilated DNA as a template were attempted according to the method of the present invention. Specifically, non-methylated cytosine in DNA of each promoter region of *Nanog* gene, *Rex1* gene, and *CD133* gene, which were stem cell marker, was uracilated, and amplification of the DNA using the DNA after uracilation as a template was carried out. In consequence, as is the case with the results of using the DNA derived from ES cell in Example 1, it was confirmed that the respective genes in the MEF cell could be amplified by the method of the present invention.

In addition, as is clear from the results of Fig. 4 to Fig. 6, it turned out that all the cytosine other than CpG dinucleotide was converted into uracil. It was presumed that the cytosine in CpG dinucleotide, which has not been converted, was methylated one. This is in accordance with many reports that cytosine of the CpG dinucleotide in a promoter region is methylated in a differentiated cell such as MEF cell. Therefore, from said result and the above-described result of Example 1 using genomic DNA extracted from ES cell, it turned out that, according to the method of the present invention, only non-methylated cytosine could be converted into uracil with high accuracy.

### Comparative Example 1: Uracilation of non-methylated cytosine in the ES cell-derived Nanog gene or ES cell-derived Rex1 gene by conventional method

To each 1 µL of solution containing ES cell-derived *Nanog* gene or ES cell-derived *Rex1* gene, 32.5 µL of sterile water, 5 µL of 10 x Gene *Taq* Universal Buffer (produced by Nippon Gene Co., Ltd.), 5 µL of dNTPs mixed solution (2.5 mmol/each) (produced by Nippon Gene Co., Ltd.), 0.5 µL of Gene *Taq* NT (produced by Nippon Gene Co., Ltd.), and each 3 µL of 5 µmol/L PCR primer Forward solution and Reverse solution for *Nanog* gene [Forward: 5' TTGTGAATTTATAGGGTTGGTGGG 3' (Nucleotide sequence 5), Reverse: 5' CAACCAAATCAACCTATCTAAAAACC 3' (Nucleotide sequence 6)], or each 3 µL of 5 µmol/L PCR primer Forward solution and Reverse solution for *Rex1* gene [Forward: 5' GGGTTATTTGAAGGGTTAGGGGTT 3' (Nucleotide sequence 7), Reverse: 5' CTTAAACCCCTCCCTTTTTAAATAA 3'(Nucleotide sequence 8)] were added to make the total volume 50 µL, and by setting the reaction at 95°C for 2 minutes → at 95°C for 20 seconds → at 55°C for 20 seconds → at 72°C for 30 seconds as 1 cycle, 35 cycles of PCR reaction were carried out. After that, the obtained PCR amplified products were fractionated by electrophoresis using 1.5% agarose gel, and whether the objective DNA was amplified was examined.

The result of the electrophoresis of *Nanog* gene was shown in Fig. 1, and the result of the electrophoresis of *Rex1* gene was shown in Fig. 2, respectively.

From said result, it turned out that sufficient quantity of the amplification product was not acquired by the conventional method. That is, it turned out that by the conventional method in which the PCR reaction is carried out after bisulfite reaction using a polymerase such as *Taq* DNA polymerase, sufficient quantity of the amplification product was not acquired.

### Comparative Example 2: Uracilation of non-methylated cytosine in the MEF cell-derived Nanog gene or MEF cell-derived Rex1 gene by conventional method

The PCR reaction was carried out by the same way as carried out in Comparative Example 1 except for using a solution containing MEF cell-derived *Nanog* gene and MEF cell-derived *Rex1* gene, which were obtained by the bisulfite reaction in Example 2 (2), instead of using a solution containing ES cell-derived *Nanog* gene and ES cell-derived *Rex1* gene. After that, the obtained PCR amplified products were fractionated by electrophoresis using 1.5% agarose gel, and whether the objective DNA was amplified was examined.

The result of electrophoresis of *Nanog* gene was shown in Fig. 1, and the electrophoresis result of *Rex1* gene was shown in Fig. 2, respectively.

From said result, it turned out that sufficient amount of amplification product has not been obtained by the conventional method. That is, even if it was a case where MEF cell-derived DNA was used, it turned out that, just as Comparative Example 1, sufficient amount of amplification product was not obtained by the conventional method.

## Claims

1. A method for obtaining DNA complementary to a single-stranded DNA, in which non-methylated cytosine has been uracilated, comprising subjecting the single-stranded DNA to 1) bisulfite reaction and 2) reverse transcriptase reaction in this order.

2. The method according to claim 1, wherein the single-stranded DNA is obtained by subjecting a double-stranded DNA to alkaline treatment.

3. A method for amplifying DNA complementary to a single-stranded DNA, in which non-methylated cytosine has been uracilated, comprising subjecting the single-stranded DNA to 1) bisulfite reaction, 2) reverse transcriptase reaction and 3) PCR reaction in this order.

4. The method according to claim 3, wherein the PCR reaction is carried out using α-type DNA polymerase as a DNA polymerase.

5. The method according to claim 3, wherein the single-stranded DNA is obtained by subjecting double-stranded DNA to alkaline treatment.

6. The method according to claim 3, wherein the PCR reaction is carried out using α-type DNA polymerase as a DNA polymerase, and the single-stranded DNA is obtained by subjecting double-stranded DNA to alkaline treatment.

7. A method for detecting methylated cytosine in a single-stranded DNA, comprising subjecting the single-stranded DNA to 1) bisulfite reaction, 2) reverse transcriptase reaction and 3) PCR reaction in this order, and carrying out nucleotide sequence analysis of the obtained PCR amplification product.

8. The method according to claim 7, wherein the PCR reaction is carried out using α-type DNA polymerase as a DNA polymerase.

9. The method according to claim 7, wherein the single-stranded DNA is obtained by subjecting double-stranded DNA to alkaline treatment.

10. The method according to claim 7, wherein the PCR reaction is carried out using α-type DNA polymerase as a DNA polymerase, and the single-stranded DNA is obtained by subjecting double-stranded DNA to alkaline treatment.


SEQUENCE LISTING

<110>  WAKO PURE CHEMICAL INDUSTRIES, LTD.

<120>  A detection method for methyl cytosine

<130>  1838JP

<160>  21

<170>  PatentIn version 3.3

<210>  1
<211>  24
<212>  DNA
<213>  Artificial

<220>
<223>  synthetic

<400>  1
ctgtgaattc acagggctgg tggg                                              24


<210>  2
<211>  26
<212>  DNA
<213>  Artificial

<220>
<223>  synthetic

<400>  2
caaccaaatc agcctatctg aaggcc                                            26


<210>  3
<211>  24
<212>  DNA
<213>  Artificial

<220>
<223>  synthetic

<400>  3
gggtcacctg aagggccagg ggcc                                              24


<210>  4
<211>  25
<212>  DNA
<213>  Artificial

<220>
<223>  synthetic

<400>  4
cttggacccc tcccttttta gatgg                                             25


<210>  5
<211>  24
<212>  DNA

<213>  Artificial

<220>
<223>  synthetic

<400>  5
ttgtgaattt atagggttgg tggg                                              24


<210>  6
<211>  26
<212>  DNA
<213>  Artificial

<220>
<223>  synthetic

<400>  6
caaccaaatc aacctatcta aaaacc                                            26


<210>  7
<211>  24
<212>  DNA
<213>  Artificial

<220>
<223>  synthetic

<400>  7
gggttatttg aagggttagg ggtt                                              24


<210>  8
<211>  25
<212>  DNA
<213>  Artificial

<220>
<223>  synthetic

<400>  8
   cttaaacccc tcccttttta aataa                                              25


<210>  9
<211>  28
<212>  DNA
<213>  Artificial

<220>
<223>  synthetic

<400>  9
gtttttaaa ttattgagtt ttgtggag                                           28


<210>  10
<211>  28
<212>  DNA
<213>  Artificial

<220>

<223>  synthetic

<400>  10
caccacaaaa ataattaaat aaaaaccc                                          28


<210>  11
<211>  22
<212>  DNA
<213>  Artificial

<220>
<223>  synthetic

<400>  11
ccagtcacga cgttgtaaaa cg                                                22


<210>  12
<211>  22
<212>  DNA
<213>  Artificial

<220>
<223>  synthetic

<400>  12
cacacaggaa acagctatga cc                                                22


<210>  13
<211>  156
<212>  DNA
<213>  Nanog

<400>  13
ctgtgaattc acagggctgg tggggcgtgg gtgccgcctg ggtgcctggg agaatagggg      60

gtgggtaggg taggaggctt gaggggggag gagcaggacc taccctttaa atctatcgcc     120

ttgagccgtt ggccttcaga taggctgatt tggttg                                156


<210>  14
<211>  156
<212>  DNA
<213>  Artificial

<220>
<223>  synthetic

<400>  14
ttgtgaattt atagggttgg tggggtgtgg gtgttgtttg ggtgtttggg agaatagggg      60

gtgggtaggg taggaggttt gaggggggag gagtaggatt tattttttaa atttattgtt     120

ttgagttgtt ggtttttaga taggttgatt tggttg                                156


<210>  15
<211>  156
<212>  DNA
<213>  Artificial

<220>
<223>  synthetic

<400>  15
ttgtgaattt atagggttgg tggggcgtgg gtgtcgtttg ggtgtttggg agaatagggg      60

gtgggtaggg taggaggttt gaggggggag gagtaggatt tattttttaa atttatcgtt     120

ttgagtcgtt ggtttttaga taggttgatt tggttg                              156


<210>  16
<211>  155
<212>  DNA
<213>  Rex1

<400>  16
gggtcacctg aagggccagg ggcccgccca catccccgcc cacacccacc ttgagcgctt      60

ctcattgggc attccagcct acccagctcg gagttagtta ctccgtaagt gtggccggaa     120

cagagttcgt ccatctaaaa agggaggggt ccaag                               155


<210>  17
<211>  155
<212>  DNA
<213>  Artificial

<220>
<223>  synthetic

<400>  17
gggttatttg aagggttagg ggtttgttta tatttttgtt tatatttatt ttgagtgttt      60

tttattgggt attttagttt atttagtttg gagttagtta ttttgtaagt gtggttggaa     120

tagagtttgt ttatttaaaa agggaggggt ttaag                               155


<210>  18
<211>  155
<212>  DNA
<213>  Artificial

<220>
<223>  synthetic

<400>  18
gggttatttg aagggttagg ggttcgttta tattttcgtt tatatttatt ttgagcgttt      60

tttattgggt attttagttt gtttagttcg gagttagtta tttcgtaagt gtggtcggaa     120

tagagttcgt ttatttaaaa agggaggggt ttaag                               155


<210>  19
<211>  240
<212>  DNA
<213>  CD133

<400>  19

gctctttaaa ttaccgagcc ttgtggagac cccggcacct ggccttaagc tcagccctga     60

gtaagtacgt ttctgaagga gtcggtgttc ctttagttcc atccccaaac ctctgccaga    120

gaccctagag gacgcaacag gttcccagag tcctggagtc ccgtggtcct gactcaggca    180

cccttagcca acccggggcg gagcagtggg acgggctctc acccaatcac tcttgtggtg    240


<210>  20
<211>  240
<212>  DNA
<213>  Artificial

<220>
<223>  synthetic

<400>  20
gttttttaaa ttattgagtt ttgtggagat tttggtattt ggttttaagt ttagttttga     60

gtaagtatgt ttttgaagga gttggtgttt ttttagtttt atttttaaat ttttgttaga    120

gattttagag gatgtaatag gtttttagag ttttggagtt ttgtggtttt gatttaggtg    180

tttttagtta atttggggtg gagtagtggg atgggttttt atttaattat ttttgtggtg    240


<210>  21
<211>  240
<212>  DNA
<213>  Artificial

<220>
<223>  synthetic

<400>  21
gttttttaaa ttattgagtt ttgtggagat ttcggtattt ggttttaagt ttagttttga     60

gtaagtatgt ttttgaagga gttggtgttt ttttagtttt atttttaaat ttttgttaga    120

gattttagag gatgtaatag gtttttagag ttttggagtt ttgtggtttt gatttaggta    180

tttttagtta atttggggtg gagtagtggg atgggttttt atttaattat ttttgtggtg    240